(19) Europäisches Patentamt
European Patent Office
Office européen des brevets

(11) Numéro de publication : 0 432 051 B1

(12) # FASCICULE DE BREVET EUROPEEN

(45) Date de publication du fascicule du brevet : **23.06.93 Bulletin 93/25**

(51) Int. Cl.$^5$ : **A61K 7/09**, A61K 7/06

(21) Numéro de dépôt : **90403486.5**

(22) Date de dépôt : **07.12.90**

(54) **Composition cosmétique réductrice pour la permanente des cheveux, à base de cystéamine et/ou de son dérivé N-acétylé et d'un polymère cationique, et son utilisation dans un procédé de déformation permanente des cheveux.**

(30) Priorité : **08.12.89 FR 8916273**
**19.01.90 FR 9000637**

(43) Date de publication de la demande : **12.06.91 Bulletin 91/24**

(45) Mention de la délivrance du brevet : **23.06.93 Bulletin 93/25**

(84) Etats contractants désignés : **AT BE CH DE DK ES FR GB GR IT LI NL SE**

(56) Documents cités :
**EP-A- 0 208 951**
**EP-A- 0 299 764**
**EP-A- 0 362 663**
**GB-A- 2 114 616**
**PATENT ABSTRACTS OF JAPAN, vol. 12, no. 405 (C-539)[3252], 26 octobre 1988; & JP-A-63 146 808 (KAO CORP.) 18-06-1988**

(73) Titulaire : **L'OREAL**
**14, Rue Royale**
**F-75008 Paris (FR)**

(72) Inventeur : **Dubief, Claude**
**9, rue E. Rostand**
**F-78150 Le Chesnay (FR)**
Inventeur : **Dupuis, Christine**
**15, rue Seveste**
**F-75018 Paris (FR)**

(74) Mandataire : **Stalla-Bourdillon, Bernard et al**
**CABINET NONY & CIE 29, rue Cambacérès**
**F-75008 Paris (FR)**

EP 0 432 051 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet une composition cosmétique réductrice pour permanente des cheveux à base de cystéamine et/ou de son dérivé N-acétylé et d'un polymère cationique et son utilisation dans un procédé de déformation permanente des cheveux à froid.

La technique classique pour réaliser la déformation permanente des cheveux consiste à réaliser, dans un premier temps, l'ouverture des ponts disulfures de la kératine à l'aide d'une composition contenant un agent réducteur (étape de réduction), puis, après avoir de préférence rincé la chevelure, à reconstituer dans un second temps lesdites liaisons disulfures en appliquant, sur les cheveux sous tension, une composition oxydante, (étape d'oxydation, dite aussi de fixation) de façon à donner aux cheveux la forme définitive désirée. Cette technique permet indifféremment de réaliser soit une ondulation des cheveux soit un défrisage ou décrêpage.

Parmi les agents réducteurs permettant de réaliser le premier temps d'une opération de permanente on utilise généralement des mercaptans tels que l'acide thioglycolique, l'acide thiolactique ou un mélange de ces acides ainsi que leurs esters par exemple le monothioglycolate de glycérol ou de glycol.

Par ailleurs, il a également été proposé d'utiliser, en tant qu'agent réducteur l'amino-2 éthanethiol ou cystéamine, à la suite des travaux de PURI et al (International Journal of Cosmetic Science 1,59-67,1979) ou EP-A-0 299 764.

Ces agents réducteurs ayant un effet détériorant sur les cheveux, il a été préconisé de les associer à des polymères cationiques.

On a toutefois observé que l'utilisation d'un certain nombre de ces polymères cationiques, associés à la cystéamine, engendrait des compositions réductrices instables dans le temps. Après diverses études, sur un très grand nombre de ces polymères cationiques, on a constaté de façon tout à fait inattendue et surprenante qu'une classe particulière de copolymères cationiques, à base de N-vinylpyrrolidone et d'acrylate ou méthacrylate de dialkyle ($C_1$-$C_4$) aminoalkyle ($C_1$-$C_{18}$) non quaternisés, conduisait non seulement à des compositions stables mais encore permettait d'obtenir des permanentes d'excellente qualité présentant notamment une bonne tenue avec une frisure plus durable dans le temps.

En outre, il s'est avéré que ces compositions réductrices étaient exemptes d'odeur ce qui est particulièrement recherché non seulement pour les personnes subissant une opération de permanente des cheveux mais également pour les personnes qui les effectuent.

Enfin les compositions réductrices selon l'invention permettent d'obtenir un meilleur effet traitant des pointes des cheveux.

La bonne stabilité des compositions réductrices selon l'invention permet donc de remédier aux inconvénients des compositions de l'art antérieur qui le plus souvent devaient être réalisées au moment de l'emploi, par mélange de l'agent réducteur d'une part et du polymère cationique d'autre part, ceux-ci étant conditionnés séparément.

La présente invention a donc pour objet une composition cosmétique réductrice pour réaliser une déformation permanente des cheveux à froid, contenant une association d'un agent réducteur et d'un polymère cationique, l'agent réducteur étant la cystéamine ou l'un de ses sels et/ou la N-acétyl cystéamine et le polymère cationique étant un copolymère de 45 à 99,5 moles % de N-vinylpyrrolidone et de 55 à 0,5 moles % d'acrylate ou méthacrylate de dialkyle ($C_1$-$C_4$) aminoalkyle ($C_1$-$C_{18}$) non quaternisé.

Selon l'invention on utilise de préférence l'agent réducteur de la composition à une concentration comprise entre 0,1 et 15 % en poids, de préférence entre 3 et 8% en poids par rapport au poids total de la composition réductrice.

Selon une forme préférée la cystéamine est utilisée sous forme de son chlorhydrate à une concentration exprimée en cystéamine base telle qu'indiquée ci-dessus.

Parmi les copolymères cationiques, répondant à la définition ci-dessus, on préfère utiliser selon l'invention les copolymères de 80 à 99,5 moles % de N-vinylpyrrolidone et de 0,5 à 20 moles % de méthacrylate de diméthylaminoéthyle.

Parmi ces derniers, ceux tout particulièrement préférés sont les copolymères vendus par la Société GAF sous les dénominations commerciales de "Copolymer 845", "Copolymer 937" et "Copolymer 958" ; le "Copolymer 845" étant tout particulièrement préféré.

Les caractéristiques de ces copolymères cationiques, à base de N-vinylpyrrolidone et de méthacrylate de diméthylaminoéthyle, sont les suivantes :

"Copolymer 845"

poids moléculaire moyen = $10^6$
vendu sous forme d'une solution aqueuse à 20% en poids ayant un pH de 6 à 8 et une viscosité de 20.000

- 40.000 mPa.s (Brookfield RV 20 rpm # 7 spindle θ 25°C).

"Copolymer 937"

poids moléculaire moyen = $10^6$

vendu sous forme d'une solution aqueuse à 20% en poids ayant un pH de 4,5 à 6,5 et une viscosité de 30.000 - 70.000 mPa.s (Brookfield RV 20 rpm # 7 spindle θ 25°C).

"Copolymer 958"

poids moléculaire moyen = $10^5$

vendu sous forme d'une solution alcoolique à 50% en poids ayant un pH de 6,2 à 6,8.

Dans les compositions réductrices selon l'invention la concentration en copolymère cationique tel que défini ci-dessus, est généralement comprise entre 0,1 et 3% en poids et de préférence entre 0,5 et 2% (exprimé en matière active) par rapport au poids total de la composition réductrice.

Selon un mode de réalisation préféré de l'invention la cystéamine ou l'un de ses sels et/ou la N-acétyl cystéamine peut être associé à de la cystéine ou de la N-acétylcystéine et dans ce cas ces dernières sont présentes dans la composition à une concentration comprise entre 0,1 et 10% en poids et de préférence entre 1,5 et 8% en poids.

De façon préférentielle la composition réductrice contient également au moins un agent tensioactif de type nonionique, anionique ou amphotère mais de préférence du type nonionique et plus particulièrement un tensioactif nonionique poly (hydroxypropyléther).

Parmi les agents tensio-actifs nonioniques poly (hydroxypropyléther) on peut notamment mentionner les composés des formules (I) à (III) ci-après et/ou les composés préparés selon les procédés décrits dans les paragraphes (3) et (4) ci-dessous :

(1)

$$R_1O-\left[C_3H_5\ (OH)\ O\right]_n-H \qquad (I)$$

dans laquelle :

le motif divalent $-C_3H_5(OH)\ O-$ représente les structures suivantes, prises en mélange ou séparément :

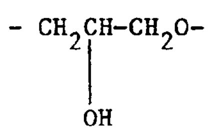 ; 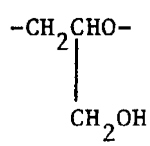 ; 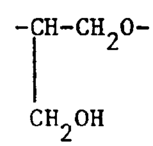

$R_1$ représente un radical ou mélange de radicaux alkyles contenant de 10 à 14 atomes de carbone et n est un nombre entier ou décimal statistique de 2 à 10 et de préférence de 3 à 6.

Les composés de formules (I) ci-dessus particulièrement préférés sont ceux dans lesquels soit $R_1$ représente le radical $C_{12}\ H_{25}$ et n = 4,2 ; soit $R_1$ représente un mélange de radicaux alkyles en $C_{10}\ H_{21}$ et $C_{12}\ H_{25}$ et n = 3,75.

Ces composés de formule (I) sont décrits dans le brevet français n° 1.477.048.

(2)

$$R_2-CONH-CH_2-CH_2-O-CH_2-CH_2-O-\left[C_3\ H_5\ (OH)\ O\right]_m-H \qquad (II)$$

dans laquelle :

le motif divalent $-C_3H_5\ (OH)\ O-$ a la même signification que ci-dessus pour la formule (I),

$R_2$ représente un radical ou un mélange de radicaux alkyles et/ou alkényles ayant de 11 à 17 atomes

de carbone et m désigne un nombre entier ou décimal statistique de 1 à 5, de préférence 1,5 à 4.

Parmi ces composés de formule (II) on préfère tout particulièrement le composé ayant la formule suivante :

$$R_2\text{-CONH-CH}_2\text{-CH}_2\text{-O-CH}_2\text{-CH}_2\text{-O}-\left[C_3\,H_5\,(OH)\,O\right]_{3,5}\text{-H}$$

dans laquelle :

le motif divalent $-C_3\,H_5(OH)\,O-$ a la même signification que ci-dessus pour la formule (I),

$R_2$ représente un mélange de radicaux alkyles et alkényles choisis parmi les radicaux $C_{11}\,H_{23}$ ou $C_{13}\,H_{27}$ et les radicaux dérivés des acides gras du coprah et de l'acide oléïque.

Ces composés de formule (II) sont plus particulièrement décrits dans le brevet français n° 76-31975 (2.328.763).

(3)

$$\begin{array}{l} R_3\text{-CH-CH}_2\text{-O}-\left[C_3\,H_5\,(OH)\,O\right]_p\,H \qquad (III)\\ \quad\;\;| \\ \quad\;\;O-\left[C_3H_5(OH)O\right]_{p'}-H \end{array}$$

dans laquelle :

le motif divalent $-C_3\,H_5\,(OH)O-$ a la même signification que ci-dessus pour la formule (I),

$R_3$ désigne un radical aliphatique, cycloaliphatique, arylaliphatique, ayant de préférence de 7 à 21 atomes de carbone et leurs mélanges, les chaines aliphatiques représentant en particulier des chaînes alkyles ayant de 1 à 6 groupements éther, thioéther et/ou hydroxyméthylène et p + p' est un nombre statistique compris entre 1 et 10 inclus.

Ces composés sont préparés par condensation en catalyse alcaline, de 2 à 10 et de préférence de 2,5 à 6 moles de glycidol sur un alpha-diol ou un mélange d'alpha-diols en $C_{10}$-$C_{14}$, à la température de 120-180°C et de préférence de 140 à 160°C.

Selon un mode particulier de réalisation, on préfère les composés préparés par condensation en catalyse alcaline de 3,5 moles de glycidol sur un mélange d'alpha-diols ayant de 10 à 14 atomes de carbone.

Ces composés de formule (III) sont obtenus selon le procédé décrit dans le brevet français n° 71-17206 (2.091.516).

(4) Composés préparés par condensation, en catalyse acide, de 2 à 10 et de préférence de 2,5 à 6 moles de glycidol par mole d'alcool ou d'alpha-diol contenant 10 à 14 atomes de carbone à une température de 50 à 120°C, le glycidol étant ajouté lentement à l'alcool ou à l'alpha-diol.

Parmi ces composés, celui obtenu par condensation de la monochlorhydrine (2,5 moles) en présence de soude sur le dodécanediol-1,2 est particulièrement préféré.

Ces composés sont préparés selon le procédé décrit dans le brevet français n° 72-40822 (2.169.787).

(5) Composés de poly (hydroxypropyléther) préparés par poly-addition de monochlorhydrine du glycérol sur un composé organique poly hydroxylé en présence d'une base forte, par élimination au fur et à mesure de l'eau par distillation.

Ces composés sont plus particulièrement décrits dans le brevet français n° 84-19267 (n° 2.574.786).

Lorsque les compositions selon l'invention contiennent au moins un agent tensioactif, celui-ci est généralement présent à une concentration comprise entre 0,5 et 10% en poids et de préférence entre 2 et 6% par rapport au poids total de la composition.

Les compositions réductrices, sont essentiellement aqueuses, et se présentent sous forme d'une lotion, épaissie ou non, d'une crème ou d'un gel. Le pH de ces compositions varie entre 5 et 10 et de préférence entre 6,5 et 9,5.

On peut utiliser comme agent acidifiant un acide tel que l'acide chlorhydrique, l'acide phosphorique ou l'acide citrique et comme agent alcalinisant un composé choisi parmi l'ammoniaque, la mono, di, ou triéthanolamine, les carbonates ou bicarbonates alcalins ou d'ammonium.

Les compositions réductrices selon l'invention peuvent également renfermer divers adjuvants cosmétiques conventionnels utilisés dans les permanentes pour cheveux tels que par exemple un agent adoucissant, un agent opacifiant, un agent séquestrant, un agent traitant, un parfum et/ou un colorant.

La présente invention a également pour objet un procédé d'ondulation des cheveux dans lequel on applique une composition réductrice telle que définie ci-dessus sur des cheveux mouillés préalablement enroulés sur des rouleaux ayant de 4 à 20 mm. de diamètre, la composition pouvant, éventuellement être appliquée au fur et à mesure de l'enroulage des cheveux; on laisse ensuite agir la composition réductrice pendant un temps de 5 à 60 min., de préférence de 5 à 30 min. puis on rince abondamment après quoi on applique sur les cheveux enroulés une composition oxydante permettant de reformer les liaisons disulfures de la kératine, pendant un temps de pose de 2 à 10 min. Après avoir enlevé les rouleaux, on rince abondamment la chevelure. Cette étape d'oxydation peut être réalisée en laissant agir l'oxygène de l'air.

La composition d'oxydation ou oxydante est du type couramment utilisé et contient comme agent oxydant par exemple de l'eau oxygénée, un bromate alcalin, un persel, un polythionate ou un mélange de bromate alcalin et d'un persel.

La concentration en eau oxygénée peut varier de 1 à 10 volumes, mais est de préférence de 8 volumes, la concentration en bromate alcalin est de 1 à 12% et celle en persel de 0,1 à 15% en poids par rapport au poids total de la composition oxydante.

Le pH de la composition oxydante peut varier entre 2 et 8 mais de préférence entre 3 et 6.

L'eau oxygénée peut être stabilisée par exemple par la phénacétine, l'acétanilide, les phosphates mono et trisodiques ou par le sulfate d'hydroxy-8 quinoléine.

L'oxydation peut être effectuée immédiatement ou être différée.

Les compositions oxydantes peuvent également contenir des agents alcalinisants ou acidifiants, des agents conservateurs, des agents séquestrants, des opacifiants et éventuellement un copolymère cationique tel que ceux définis ci-dessus pour la composition réductrice.

La présente invention à également pour objet un procédé de défrisage ou de décrêpage des cheveux dans lequel on applique sur les cheveux une composition réductrice selon l'invention, puis l'on soumet les cheveux à une déformation mécanique permettant de les fixer dans leur nouvelle forme, par une opération de lissage des cheveux avec un peigne à large dents, avec le dos d'un peigne ou à la main. Après un temps de pose de 5 à 60 min., en particulier de 5 à 30 min., on procède alors à un nouveau lissage puis on rince soigneusement et on applique la composition oxydante ou fixatrice que l'on laisse agir pendant 2 à 10 min. environ puis on rince abondamment les cheveux.

ETUDES COMPARATIVES

De façon à mettre en évidence les bonnes propriétés des compositions réductrices selon l'invention on a comparé la composition suivante :

Composition A:

- Chlorhydrate de cystéamine 5 g
- Copolymère de N-vinylpyrrolidone/méthacrylate de diméthylaminoéthyle, en solution à 20% en poids, vendu par la Société GAF sous la dénomination "Copolymer 845" 1 g
- $NH_4OH$ qs pH = 9
- Eau qsp 100 g

à des compositions réductrices identiques à la composition A mais dans lesquelles le "Copolymer 845" a été remplacé par la même quantité (d'un polymére cationique différent, ces compositions étant les suivantes :

Composition B: "GAFQUAT 734" vendu par la Société GAF (Copolymére N-vinyl pyrrolidone/méthacrylate de diméthylaminoéthyle quaternisé de PM = 100.000).

Composition C: "GAFFIX VC 713" vendu par la Société GAF (Terpolymère N-vinyl pyrrolidone/vinyl caprolactame/méthacrylate de diméthylaminoéthyle).

Composition D: "MERQUAT 100" vendu par la société MERCK-CALGON (USA) (Homopolymère de chlorure de diméthyldiallylammonium de PM < 100.000).

Composition E: Polymère cationique décrit et préparé selon le brevet US n° 4.217.914, constitué de motifs récurrents de formule :

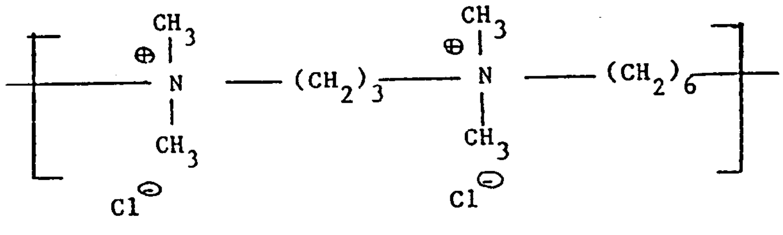

Les compositions A à E ainsi qu'une composition témoin (composition ne contenant pas de copolymère cationique) ont été soumises à deux tests différents : (i) le premier ayant porté sur l'amélioration en % du rendement de la frisure selon la méthode de la planche à chevilles dite "Pegboard" dont la technique, mise au point par Kirby et al, a été décrite dans " The Chemistry and Manufacture of Cosmetics" vol.IV, 2ème édition, Ed. G. de Navarre, p. 1211-1216, 1975 et (ii) le second ayant porté sur la tenue de la frisure dans le temps par la mesure de la chute de la frisure exprimée en cm dans une atmosphère à degré d'humidité relative constante de 90%.

Les résultats selon ces deux tests, réalisés en double essais, sont rassemblés dans le tableau suivant :

| TESTS | COMPOSITION TEMOIN | COMPOSITION A | COMPOSITIONS DE COMPARAISON B | C | D | E |
|---|---|---|---|---|---|---|
| Amélioration en % du Rdt de la frisure | -- | 15-20 | 0 | 0 | 0 | 0 |
| Tenue de la frisure dans le temps exprimée par la chute de l'ondulation en cm | | | | | | |
| Après 1 h 30 | 3,3 | 1,2 | 2,3 | 3,5 | 3,4 | 4 |
| Après 4 jours | 5,5 | 2 | 5,4 | 5 | 5,4 | 6 |

On peut constater d'après ces résultats que la composition A selon l'invention, conduit à une amélioration relative du rendement de la frisure de 15 à 20 % par rapport à la composition témoin. Les autres compositions B à E ne conduisent à aucune amélioration du rendement de frisure de la permanente. Par ailleurs, en ce qui concerne la tenue de la frisure dans le temps, celle-ci est nettement supérieure pour la composition A que pour les compositions B à E aussi bien après 1h 30 qu'après 4 jours.

On va maintenant donner à titre d'illustration et sans aucun caractère limitatif plusieurs exemples de compositions réductrices selon l'invention ainsi que des procédés pour sa mise en oeuvre.

EXEMPLES DE COMPOSITION

EXEMPLE 1:

On réalise une ondulation permanente des cheveux en appliquant sur l'ensemble de la chevelure, la composition réductrice suivante :

- Chlorhydrate de cystéamine 4 g
- Copolymère de N-vinylpyrrolidone / méthacrylate de diméthylaminoéthyle en solution aqueuse à 20 % en poids vendu par la société GAF sous la dénomination de "Copolymer 845" 0,5 g
- Cocoamidopropyl-Bétaine vendue par la société GOLDSCHMIDT sous la dénomination de "Tégobétaïne HS" 2 g
- Ammoniaque qs pH=9,2

- Colorant qs,
- Parfum qs
- Eau .qsp 100 g

On enroule ensuite les cheveux sur des rouleaux puis on laisse agir la composition pendant un temps de 10 à 20 min. selon la nature des cheveux.

Après rinçage à l'eau, on applique ensuite sur les cheveux réduits, la composition oxydante suivante :

- Bromate de sodium : 8 g
- Phosphate mono et trisodique : 0,8 g
- Triéthanolamine qs pH=7,5
- Parfum qs
- Colorant qs
- Eau .qsp 100 g

On laisse agir la composition oxydante pendant un temps de 10 à 15 min., on rince les cheveux à l'eau puis on enlève alors les rouleaux et on procède à un séchage.

Les cheveux présentent une excellente tenue de la frisure au cours du temps.

Selon le même mode opératoire que celui décrit à l'Exemple 1 ci-dessus, on a également réalisé des ondulations permanentes des cheveux à l'aide des compositions réductrices et oxydantes suivantes :

EXEMPLE 2:

Composition réductrice:

- Chlorhydrate de cystéamine : 8 g
- Copolymère de N-vinylpyrrolidone / méthacrylate de diméthylaminoéthyle en solution aqueuse à 20 % en poids vendu par la société GAF sous la dénomination de "Copolymer 845" 2 g
- Tensioactif nonionique poly (hydroxypropyléther) préparé par condensation, en catalyse alcaline, de 3,5 moles de glycidol sur un mélange d'alphadiols ayant 10 à 14 atomes de carbone, selon le procédé décrit dans le brevet FR n° 71-17206 (2.091.516) 3 g
- Monoéthanolamine qs pH=9,0
- Parfum qs
- Colorant qs
- Eau .qsp 100 g

On laisse agir la composition réductrice pendant un temps de 10 à 20 min.

Composition oxydante:

- Peroxyde d' hydrogène en solution aqueuse à 200 volumes 4,8 g
- Stabilisants : 0,05 g
  (Sulfate d'hydroxy-8 quinoléine - phénacétine)
- Acide citrique qs pH=3,0
- Parfum qs
- Colorant qs
- Eau .qsp 100 g

On laisse agir la composition oxydante pendant 10 min.

EXEMPLE 3

Composition réductrice:

- Chlorhydrate de cystéamine 10 g
- Copolymère de N-vinylpyrrolidone / méthacrylate de diméthylaminoéthyle en solution dans l'éthanol à 50 % en poids vendu par la société GAF sous la dénomination de "Copolymer 958" 1 g
- Tensioactif nonionique poly (hydroxypropyléther) préparé par condensation, en catalyse alcaline, de 3,5 moles de glycidol sur un mélange d'alphadiols ayant 11 à 14 atomes de carbone, selon le procédé décrit dans le brevet FR n° 71-17206 (2.091.516) : 4 g
- Ammoniaque qs pH=8,5
- Colorant qs
- Parfum qs

- Eau .qsp 100 g

Composition oxydante:

- Peroxyde d' hydrogène en solution aqueuse à 200 volumes 4,8 g
- Stabilisants : 0,05 g
  (Sulfate d'hydroxy-8 quinoléine - phénacétine)
- Acide citrique qs pH=3,0
- Parfum qs
- colorant qs
- Eau .qsp 100 g

On laisse agir la composition oxydante pendant 10 min.

EXEMPLE 4

Composition réductrice :

- Chlorhydrate de cystéamine 5 g
- Copolymère de N-vinylpyrrolidone/méthacrylate de diméthylaminoéthyle en solution aqueuse à 20 % en poids vendu par la Société GAF sous la dénomination de "Copolymer 937" 2 g
- Cocoamidopropyl - Bétaïne vendue par la Société GOLDSCHMIDT sous la dénomination de "Tégobétaïne HS" 2 g
- Ammoniaque qsp pH = 9,0
- Colorant qs
- Parfum, conservateur qs
- Eau qsp 100 g

Composition oxydante :

- peroxyde d'hydrogène en solution aqueuse à 200 volumes 4,8 g
- Stabilisants : 0,05 g
  (sulfate d'hydroxy-8 quinoléine - phénacétine)
- Acide nitrique qsp pH = 3,0
- Parfum qs
- Colorant qs
- Eau qsp 100 g

On laisse agir la composition oxydante pendant 10 minutes.

EXEMPLE 5

Composition réductrice :

- Chlorhydrate de cystéamine 6 g
- Cystéine 3 g
- Copolymère de N-vinylpyrrolidone/méthacrylate de diméthylaminoéthyle en solution aqueuse à 20 % en poids vendu par la Société GAF sous la dénomination de "Copolymer 845" 1 g
- Tensioactif nonionique poly (hydroxypropyléther) préparé par condensation, en catalyse alcaline, de 3,5 moles de glycidol sur un mélange d'alphadiols ayant 10 à 14 atomes de carbone, selon le procédé décrit dans le brevet FR n° 71-17206 (2.091.516) 2 g
- Ammoniaque q.s.p. pH = 8,5
- Parfum qs
- Colorant, conservateur, qs
- Eau qsp 100 g

On laisse agir la composition réductrice pendant un temps de 10 à 20 minutes.

Composition oxydante :

- Peroxyde d' hydrogène en solution aqueuse à 200 volumes 4,8 g

- Stabilisants : 0,05 g
  (sulfate d'hydroxy-8 quinoléine - phénacétine)
- Acide citrique q.s.p. pH = 3,0
- Parfum q.s.
- Colorant q.s.
- Eau q.s.p. 100 g

On laisse agir la composition oxydante pendant 10 minutes.

EXEMPLE 6

Composition réductrice :

- N-acétylcystéamine 8 g
- Copolymère de vinylpyrrolidone/méthacrylate de diméthylaminoéthyle en solution aqueuse à 20 % en poids vendu par la Société GAF sous la dénomination de "Copolymer 845" : 1 g
- Cocoamidopropyl Bétaïne vendue par la Société GOLDSCHMIDT sous la dénomination de "Tégobétaïne HS" 3 g
- Monoéthanolamine qs pH 7,0
- Colorant qs
- Parfum qs
- Eau qsp 100 g

Composition oxydante :

- Peroxyde d'hydrogène en solution aqueuse à 200 volumes 4,8 g
- Stabilisants 0,05g
  (sulfate d' hydroxy-8 quinoléïne-phénacétine)
- Acide citrique qs pH = 3,0
- Parfum qs
- Colorant qs
- Eau qsp 100 g

EXEMPLE 7

Composition réductrice :

- N-acétylcystéamine 6 g
- Copolymère de vinylpyrrolidone/méthacrylate de diméthylaminoéthyle en solution aqueuse à 20 % en poids vendu par la Société GAF sous la dénomination de "Copolymer 845" 0,5 g
- Tensio-actif non ionique poly (hydroxypropyléther) préparé par condensation, en catalyse alcaline, de 3,5 moles de glycidol sur un mélange d'alphadiols ayant 11 à 14 atomes de carbone, selon le procédé décrit dans le brevet français n° 71.17206 (2.091.516) 2 g
- Ammoniaque qs pH = 9,0
- Parfum qs
- Colorant qs
- Eau qsp 100 g

On laisse agir la composition réductrice pendant un temps de 10 à 20 min.

Composition oxydante :

- Peroxyde d'hydrogène en solution aqueuse à 200 volumes 4,8 g
- Stabilisants: 0,05 g
  (sulfate d' hydroxy-8 quinoléïne-phénacétine)
- Acide citrique qs pH = 3,0
- Parfum qs
- Colorant qs
- Eau qsp 100 g

On laisse agir la composition oxydante pendant 10 min.

EXEMPLE 8

Composition réductrice :

- N-acétylcystéamine : 6 g
- N-acétylcystéine 6 g
- Copolymère de vinylpyrrolidone/méthacrylate de diméthylaminoéthyle en solution aqueuse à 20 % en poids vendu par la Société GAF sous la dénomination de "Copolymer 845" 0,8g
- Tensio-actif non ionique poly(hydroxypropyléther)préparé par condensation, en catalyse alcaline, de 3,5 moles de glycidol sur un mélange d'alphadiols ayant 11 à 14 atomes de carbone, selon le procédé décrit dans le brevet français n° 71.17206 (2.091.516) 4 g
- Ammoniaque qs pH = 9,0
- Colorant qs
- Parfum qs
- Eau qsp 100 g

On laisse agir la composition réductrice pendant un temps de 10 à 20 min.

Composition oxydante

- Peroxyde d'hydrogène en solution aqueuse à 200 volumes 4,8g
- Stabilisants : 0,05g
  (sulfate d'hydroxy-8 quinoléine-phénacétine)
- Acide citrique qs pH = 3,0
- Parfum qs
- Colorant qs
- Eau qsp 100 g

On laisse agir la composition oxydante pendant 10 min.

EXEMPLE 9

Composition réductrice :

- Chlorhydrate de cystéamine 12 g
- N-acétyl cystéine 2 g
- Copolymère de N-vinylpyrrolidone/méthacrylate de diméthylaminoéthyle en solution aqueuse à 20 % en poids vendu par la Société GAF sous la dénomination de "Copolymer 845" 1,5 g
- Ammoniaque q.s.p pH = 9,0
- Colorant qs
- Parfum qs
- Conservateur qs
- Eau q.s.p 100 g

Composition oxydante :

- Peroxyde d'hydrogène en solution aqueuse à 200 volumes 4,8 g
- Acide citrique q.s.p pH = 3,0
- Stabilisants q.s.
- Eau q.s.p 100 g

EXEMPLE 10

Composition réductrice

- N-Acétyl cystéamine 10 g
- Cystéine 2 g
- Copolymère de N-vinylpyrrolidone/méthacrylate de diméthylaminoéthyle en solution aqueuse à 20 % en poids vendu par la Société GAF sous la dénomination de "Copolymer 845" 0,4 g
- Ammoniaque q.s.p. pH = 9,0

- Colorant q.s.
- Parfum q.s.
- Conservateur q.s.
- Eau q.s.p 100 g

Composition oxydante

- Peroxyde d'hydrogène en solution aqueuse à 200 volumes 4,8 g
- Acide citrique q.s.p pH = 3,0
- Stabilisants q.s.
- Eau q.s.p 100 g

## Revendications

1. Composition cosmétique réductrice pour réaliser une déformation permanente des cheveux à froid, contenant une association d'un agent réducteur et d'un polymère cationique, caractérisé par le fait que l'agent réducteur est la cystéamine ou l'un de ses sels et/ou la N-acétylcystéamine et que le polymère cationique est un copolymère de 45 à 99,5 moles % de N-vinylpyrrolidone et de 55 à 0,5 moles % d'acrylate ou méthacrylate de dialkyle ($C_1$-$C_4$) aminoalkyle ($C_1$-$C_{18}$) non quaternisé.

2. Composition selon la revendication 1 caractérisée par le fait que le sel de cystéamine est le chlorhydrate de cystéamine.

3. Composition selon la revendication 1 ou 2 caractérisée par le fait que l'agent réducteur est présent à une concentration, comprise entre 0,1 et 15% en poids et de préférence entre 3 et 8% en poids par rapport au poids total de la composition.

4. Composition selon l'une quelconque des revendications 1 à 3 caractérisée par le fait que le copolymère cationique est un copolymère de 80 à 99,5 moles % de N-vinylpyrrolidone et de 0,5 à 20 moles % de méthacrylate de diméthylaminoéthyle.

5. Composition selon l'une quelconque des revendications précédentes caractérisée par le fait que la concentration en copolymère cationique est comprise entre 0,1 et 3% en poids et de préférence entre 0,5 et 2% (exprimé en matière active) par rapport au poids total de la composition.

6. Composition selon l'une quelconque des revendications précédentes caractérisée par le fait que l'agent réducteur est associé à de la cystéine ou de la N-acétylcystéine, ces dernières étant présentes à une concentration comprise entre 0,1 et 10% et de préférence entre 1,5 et 8% en poids par rapport au poids total de la composition.

7. Composition selon l'une quelconque des revendications précédentes caractérisée par le fait que la composition réductrice contient au moins un agent tensioactif de type nonionique, anionique ou amphotère.

8. Composition selon la revendication 7 caractérisée par le fait que l'agent tensioactif est du type nonionique poly (hydroxypropyléther).

9. Composition selon l'une quelconque des revendications 7 ou 8, caractérisée par le fait que l'agent tensioactif est présent à une concentration comprise entre 0,5 et 10% en poids et de préférence entre 2 et 6% par rapport au poids total de la composition.

10. Composition selon l'une quelconque des revendications précédentes caractérisée par le fait que son pH est compris entre 5 et 10 et de préférence 6,5 et 9,5.

11. Composition selon l'une quelconque des revendications précédentes caractérisée par le fait qu'elle contient en outre au moins un adjuvant cosmétique choisi parmi un agent adoucissant, un agent opacifiant, un agent sequestrant, un agent traitant, un parfum et/ou un colorant.

12. Procédé de déformation permanente des cheveux consistant dans une première étape à réduire les liaisons disulfures de la kératine par application sur les cheveux d'une composition réductrice, puis dans une seconde étape à reformer lesdites liaisons par application d'une composition oxydante, caractérisé par le fait que l'étape de réduction est réalisée à l'aide d'une composition cosmétique réductrice telle que revendiquée selon l'une quelconque des revendications 1 à 12.

**13.** Procédé selon la revendication 12 pour l'ondulation des cheveux, caractérisé par le fait que la composition réductrice est appliquée sur des cheveux mouillés, enroulés sur des rouleaux ayant de 4 à 20mm de diamètre.

**14.** Procédé selon la revendication 12 pour le décrêpage ou le défrisage des cheveux, caractérisé par le fait qu'après application sur les cheveux de la composition réductrice, ceux-ci sont soumis à une opération de lissage à l'aide d'un peigne.

**15.** Procédé selon l'une quelconque des revendications 12 à 14 caractérisé par le fait que l'on laisse agir la composition réductrice pendant un temps compris entre 5 et 60 minutes.

## Patentansprüche

**1.** Reduzierende kosmetische Zusammensetzung zur Durchführung einer dauerhaften Verformung des Haares auf kaltem Wege, enthaltend eine Verbindung eines Reduktionsmittels und eines kationischen Polymers, dadurch gekennzeichnet, daß das Reduktionsmittel Cysteamin oder eines seiner Salze und/oder N-Acetylcysteamin ist, und daß das kationische Polymer ein Copolymer aus 45 bis 99,5 Mol-% N-Vinylpyrrolidon und 55 bis 0,5 Mol-% nicht quaternisiertes Dialkyl-($C_1$-$C_4$)-Aminoalkyl ($C_1$-$C_{18}$)-Acrylat oder Methacrylat ist.

**2.** Zusammensetzung nach Anspruch 1, dadurch gekennzeichnet, daß das Salz des Cysteamins das Chlorhydrat des Cysteamins ist.

**3.** Zusammensetzung nach Anspruch 1 oder 2, dadurch gekennzeichnet, daß das Reduktionsmittel in einer Konzentration zwischen 0,1 und 15 Gew.-% und vorzugsweise zwischen 3 und 8 Gew.-%, bezogen auf das Gesamtgewicht der Zusammensetzung, vorhanden ist.

**4.** Zusammensetzung nach einem der vorhergehenden Ansprüche 1 bis 3, dadurch gekennzeichnet, daß das kationische Polymer ein Copolymer aus 80 bis 99,5 Mol-% N-vinylpyrrolidon und 0,5 bis 20 Mol-% Dimethylaminoethylmethacrylat ist.

**5.** Zusammensetzung nach einem der vorhergehenden Ansprüche, dadurch gekennzeichnet, daß die Konzentration des kationischen Copolymers zwischen 0,1 und 3 Gew.-% und vorzugsweise zwischen 0,5 und 2 Gew.-% (ausgedrückt als aktives Material), bezogen auf das Gesamtgewicht der Zusammensetzung, beträgt.

**6.** Zusammensetzung nach einem der vorhergehenden Ansprüche, dadurch gekennzeichnet, daß das Reduktionsmittel mit dem Cystein oder dem N-Acetylcystein verbunden ist, wobei die letzteren in einer Konzentration zwischen 0,1 und 10 Gew.-% und vorzugsweise 1,5 und 8 Gew.-%, bezogen auf das Gesamtgewicht der Zusammensetzung, vorhanden sind.

**7.** Zusammensetzung nach einem der vorhergehenden Ansprüche, dadurch gekennzeichnet, daß die reduzierende Zusammensetzung mindestens eine nichtionisches, anionisches oder amphoteres oberflächenaktives Mittel enthält.

**8.** Zusammensetzung nach Anspruch 7, dadurch gekennzeichnet, daß das oberflächenaktive Mittel ein nichtionischer Poly(hydroxypropylether) ist.

**9.** Zusammensetzung nach einem der Ansprüche 7 oder 8, dadurch gekennzeichnet, daß das oberflächenaktive Mittel in einer Konzentration zwischen 0,5 und 10 Gew.-% und vorzugsweise zwischen 2 und 6 Gew.-%, bezogen auf das Gesamtgewicht der Zusammensetzung, vorhanden ist.

**10.** Zusammensetzung nach einem der vorhergehenden Ansprüche, dadurch gekennzeichnet, daß der pH-Wert zwischen 5 und 10 und vorzugsweise 6,5 und 9,5 liegt.

11. Zusammensetzung nach einem der vorhergehenden Ansprüche, dadurch gekennzeichnet, daß sie weiterhin mindestens einen kosmetischen Zusatzstoff ausgewählt aus Glättungsmittel, Opazifierungsmittel, Sequistriermittel, Behandlungsmittel, Parfum und/oder Farbstoff enthält.

12. Verfahren zur permanenten Verformung des Haars, bei dem man in einem ersten Schritt die Disulfitbindungen des Keratins durch Aufbringen einer reduzierenden Zusammensetzung auf das Haar reduziert, dann in einem zweiten Schritt die Bindungen durch Aufbringen einer oxidierenden Zusammensetzung wiederherstellt, dadurch gekennzeichnet, daß man den Schritt der Reduktion mit einer reduzierenden kosmetischen Zusammensetzung nach einem der Ansprüche 1 bis 12 durchführt.

13. Verfahren nach Anspruch 12 zur Ondulierung des Haars, bei dem man die reduzierende Zusammensetzung auf das angefeuchtete Haar aufbringt und dieses zu Rollen mit einem Durchmesser von 4 bis 20 mm aufrollt.

14. Verfahren nach Anspruch 12 zur Glättung oder Entkrausung des Haars, dadurch gekennzeichnet, daß man nach Aufbringen der reduzierenden Zusammensetzung auf die Haare, diese mit Hilfe eines Kammes glättet.

15. Verfahren nach einem der Ansprüche 12 bis 14, dadurch gekennzeichnet, daß man die reduzierende Zusammensetzung für einen Zeitraum zwischen 5 und 60 Minuten einwirken läßt.

## Claims

1. Cosmetic reducing composition for carrying out a permanent-reshaping of hair in the cold state, containing a combination of a reducing agent and a cationic polymer, characterised in that the reducing agent is cysteamine or one of its salts and/or N-acetylcysteamine, and in that the cationic polymer is a copolymer of 45 to 99.5 mol% of N-vinylpyrrolidone and 55 to 0.5 mol% of unquaternised di($C_1$-$C_4$alkyl)amino($C_1$-$C_{18}$alkyl) acrylate or methacrylate.

2. Composition according to Claim 1, characterised in that the cysteamine salt is cysteamine hydrochloride.

3. Composition according to Claim 1 or 2, characterised in that the reducing agent is present at a concentration of between 0.1 and 15 % by weight, and preferably between 3 and 8 % by weight, relative to the total weight of the composition.

4. Composition according to any one of Claims 1 to 3, characterised in that the cationic copolymer is a copolymer of 80 to 99.5 mol% of N-vinylpyrrolidone and 0.5 to 20 mol% of dimethylaminoethyl methacrylate.

5. Composition according to any one of the preceding claims, characterised in that the concentration of cationic copolymer is between 0.1 and 3 % by weight, and preferably between 0.5 and 2 % (expressed as active substance), relative to the total weight of the composition.

6. Composition according to any one of the preceding claims, characterised in that the reducing agent is combined with cysteine or N-acetylcysteine, these latter being present at a concentration of between 0.1 and 10 %, and preferably between 1.5 and 8 %, by weight relative to the total weight of the composition.

7. Composition according to any one of the preceding claims, characterised in that the reducing composition contains at least one nonionic, anionic or amphoteric type surfactant.

8. Composition according to Claim 7, characterised in that the surfactant is of the nonionic poly(hydroxypropyl ether) type.

9. Composition according to either of Claims 7 and 8, characterised in that the surfactant is present at a concentration of between 0.5 and 10 % by weight, and preferably between 2 and 6 %, relative to the total weight of the composition.

10. Composition according to any one of the preceding claims, characterised in that its pH is between 5 and 10, and preferably 6.5 and 9.5.

**11.** Composition according to any one of the preceding claims, characterised in that it contains, in addition, at least one cosmetic adjuvant chosen from a demulcent agent, an opacifying agent, a sequestering agent, a treating agent, a perfume and/or a colorant.

**12.** Process for the permanent-reshaping of hair, consisting, in a first step, in reducing the disulphide bonds of the keratin by application of a reducing composition to the hair, and, in a second step, in re-forming the said bonds by application of an oxidising composition, characterised in that the reduction step is carried out using a cosmetic reducing composition as claimed in any one of Claims 1 to 12.

**13.** Process according to Claim 12 for the waving of hair, characterised in that the reducing composition is applied to wet hair curled on rollers from 4 to 20 mm in diameter.

**14.** Process according to Claim 12 for the straightening or uncurling of hair, characterised in that, after application of the reducing composition to the hair, the latter is subjected to a smoothing operation using a comb.

**15.** Process according to any one of Claims 12 to 14, characterised in that the reducing composition is allowed to act for a time of between 50 and 60 minutes.